(19) Europäisches Patentamt
European Patent Office
Office européen des brevets

(11) Publication number : 0 244 962 B1

(12) EUROPEAN PATENT SPECIFICATION

(45) Date of publication of patent specification : 06.03.91 Bulletin 91/10

(51) Int. Cl.$^5$ : A01N 37/00, C07C 331/04, C07F 7/08

(21) Application number : 87303086.0

(22) Date of filing : 09.04.87

# (54) Substituted alkynic thiocyanates, biocidal compositions containing them and uses of such compositions.

(30) Priority : 24.04.86 GB 8610076

(43) Date of publication of application : 11.11.87 Bulletin 87/46

(45) Publication of the grant of the patent : 06.03.91 Bulletin 91/10

(84) Designated Contracting States : BE CH DE ES FR GB GR IT LI LU NL SE

(56) References cited :
FR-A- 1 471 952
CHEMICAL ABSTRACTS, vol. 71, September 29, 1969, page 365, ref. no. 60581r; Columbus, Ohio, US V. SUTORIS et al.: "Synthesis of new substances from ethynyl methyl vinyl carbinol and study of their herbicidal activity."
CHEMICAL ABSTRACTS, vol. 58, June 24, 1963, column 13981 a-c; Columbus, Ohio, US N.N.KOMAROV et al.: "Synthesis and transformations of unsaturated organosilicon compounds. VI. Synthesis and some transformations of organosilicon acetylenic mercaptans."
CHEMICAL ABSTRACTS, vol. 66, June 5, 1967, page 9791, ref. no. 104774j;Columbus, Ohio, US J:S:ADAMS Jr. et al.: "Synthesis of 4-iodo- and 4-thiocyanatobutynyl carbamates".
CHEMICAL ABSTRACTS, Vol. 71, August 18, 1969, page 244, ref.no. 30021r; Columbus, Ohio,US I.N.AZERBAEV et al.: "Chloro and thiocyanato derivatives of acetylene gamma-glycols."
CHEMICAL ABSTRACTS SERVICE; Registry Handbook, number section 1965-1971, page 2479R; Am. Chem. Soc.,US
CHEMICAL ABSTRACTS, vol. 64, January 17,1966, column 2122b-c; Columbus, Ohio, US N.V.KOMAROV et al.: "Synthesis and some transformations of primary gamma-silaacetylenic mercaptans."

(56) References cited :
CHEMICAL AND PHARMACEUTICAL BULLETIN, vol. 10, no. 11, 1962, chapter 178, pages 1094-1098; Tokyo, JP Y.YURA: "Studies on acetylenic compounds. XXVI. Ring Closure. (6). New synthetic method of heterocyclic compounds form alpha-substituted acetylenic compounds."
RECUEIL DES TRAVAUX CHIMIQUES DES PAYS-BAS, vol. 93, 1974, pages 26-29; The Hague, NL J.MEIJER et al.: "Chemistry of acetylenic ethers 104. Thermal rearrangement of 1-alkynyl 2 alkynyl sulfides in the presence of dialkylamines or dialkylphosphines. A new type of thio-claisen rearrangement.
CHEMICAL ABSTRACTS, vol. 71, July 21, 1969, page 243, ref. no. 12238n; Columbus, Ohio, US R.V. VIZGERT et al.: "Reactions of unsaturated esters of aromatic sulfonic acids." II. Alkylating properties of unsaturated esters of aromatic sulfonic acids."
CHEMICAL ABSTRACTS, vol. 105, October 20, 1986, page 92, ref. no. 135638w; Columbus, Ohio, US & JP-A-61 34 078 (NIPPON SYNTHETIC CHEM. IND.CO., LTD) 18-02-1986.

(73) Proprietor : IMPERIAL CHEMICAL INDUSTRIES PLC
Imperial Chemical House, Millbank
London SW1P 3JF (GB)

(72) Inventor : Austin, Peter William
45 Randale Drive
Bury BL9 8NF Lancashire (GB)

(74) Representative : James, David Gomer et al
Imperial Chemical Industries PLC Legal Department: Patents Po Box 6 Bessemer Road
Welwyn Garden City Herts, AL7 1HD (GB)

EP 0 244 962 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a class of compounds, some of which are new compounds, which are useful as industrial biocides.

More particularly this invention relates to a class of propargyl thiocyanate compounds, many of which have both antibacterial and antifungal properties, and which are useful as industrial biocides. Industrial biocides are useful to prevent industrial spoilage, in particular that caused by bacteria and fungi. Thus, such materials are useful in the preservation of paints, lattices, adhesives, leather, wood, metal working fluids and cooling water.

Propargyl compounds having biological, for example phytological, activity are known and are disclosed for example in UK Patent No.1236969 and US Patent 3075938, US Patent 3226426 and Canadian Patent 627647.

Iodopropargyl compounds are known which exhibit good antifungal properties but which are mediocre antibacterials, for example the carbamate derivative having the formula $I-C=CCH_2OCONHC_4H_9$ (see UK Patent 1455043) and known as Troysan Polyphase Antimildew (hereinafter referred to as "Troysan PPA"), is a commercial fungicide but lacks effective antibacterial activity.

Thiocyanate propargyl derivatives are reported in Ber (1873) 6, 729 and Chem.Pharm.Bull.Jap. (1962) 10, 1094. A compound having the formula $CH_3-C\equiv CCH_2SCN$ is reported in Rec.Trav.Chim. (1974) 93 29. The compound $C_6H_5-C\equiv CCH_2SCN$ appears to have been formed as an intermediate in Chem.Pharm.Bull.Jap. (1962) 10, 1094 but is not characterised. No antimicrobial activity has been reported in the above-mentioned literature. Other references to thiocyanates containing propargyl groups can be found in UK Patent 1210781 (to Ciba-Geigy) and UK Patent 1164486 (to BASF).

It has now been found that certain propargyl thiocyanates are surprisingly active against both bacteria and fungi. Antimicrobial properties have not been reported for this class of propargyl derivatives. Some compounds of this type are novel.

According to the present invention, there is provided a biocide composition which contains at least one compound of the formula :

$$R^1 - C \equiv C - \underset{R^3}{\overset{R^2}{\underset{|}{\overset{|}{C}}}} - SCN$$

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom, a hydrocarbyl group or a substituted hydrocarbyl group.

If the group $R^1$ is a hydrocarbyl group it can be an alkyl, cycloalkyl, aralkyl or aryl group. If $R^1$ is a hydrocarbyl group, typically the group contains no more than 20 carbon atoms, for example not more than 10 carbon atoms. We have found that compounds in which $R^1$ is hydrogen or a hydrocarbyl group have particularly good anti-fungal activity and some compounds of this type also show useful anti-bacterial activity, such compounds including those in which $R^1$ is hydrogen, phenyl or lower alkyl, that is an alkyl group containing not more than 5 carbon atoms.

If the group $R^1$ is a substituted hydrocarbyl group it can be a substituted alkyl, cycloalkyl, aralkyl or aryl group. The substituents can be, inter alia, hydrocarbyl groups, ester (that is acyloxy) groups, halogen atoms, nitrile groups or ether groups, which may themselves be substituted. If the group $R^1$ is substituted with halogen atoms, it can contain more than one halogen atom, for example as in a trifluoromethyl group.

In the group $-SiR^2R^3R^4$, it is preferred that at least one of the groups $R^2$, $R^3$ and $R^4$ is a hydrocarbyl group, for example as in a trimethylsilyl group.

Preferred compositions in accordance with the present invention are those in which $R^1$ is a group $-CH_2SCN$ or is, or contains, a halogen atom. The preferred compositions have a combination of particularly good anti-bacterial activity and anti-fungal activity. Especially preferred compositions are those in which $R^1$ is a halogen atom, most especially bromine or iodine.

The compositions of the present invention provide good wet state preservation making the compositions advantageous for use as a cutting fluid preservative and also in cooling water applications. Wood and leather preservations is another advantageous field of application of the compositions. The compositions

of the present invention can also be incorporated into paint, as paint film fungicide that can be used, in contrast with Troysan PPA, without addition of a bactericide.

The propargyl derivatives which are present in biocide composition of the present invention are soluble in many polar solvents, although the solubility is dependent on the nature of the groups $R^1$, $R^2$, $R^3$ and $R^4$. However, many of the compounds are soluble in water, alcohols, ethers, ketones and other polar solvents or mixtures thereof.

The compositions of the present invention may consist only of the propargyl derivative. However, typically the composition comprises the propargyl derivative as a solution, suspension or emulsion in a suitable liquid medium such as water. The composition may comprise a suspension or emulsion of the propargyl derivative, or a solution thereof, in a liquid medium in which the propargyl derivative is insoluble.

The composition may be incorporated into the medium to be protected using any suitable mixing technique. The composition is incorporated into the medium to be protected in an amount to provide from 0.0001 to 5% by weight of the propargyl derivative relative to the total composition. If the composition is being used to preserve a solid substrate such as leather or wood, the composition may be applied directly to the substrae or may be incorporated into a coating composition such as a paint, varnish or lacquer which is then applied to the substrate. Alternatively, the solid material may be impregnated with the composition of the present invention.

The compositions of the present invention can be used for the treatment of various media to inhibit the growth of micro-organisms.

Thus, as a further aspect of the present invention there is provided a method for inhibiting the growth of micro-organisms on, or in, a medium which comprises treating the medium with a propargyl derivative as hereinbefore defined.

The propargyl derivatives can be used in conditions in which micro-organisms grow and cause problems such as, for example, in aqueous environments including cooling water systems, paper mill liquors, metal working fluids, geological drilling lubricants, polymer emulsions, and emulsion paints. The propargyl derivatives can also be used to impregnate solid materials such as wood or leather or can be coated onto the surfaces thereof directly or incorporated into a paint, varnish or lacquer.

As a yet further aspect of the present invention there are provided new propargyl derivatives of the formula :

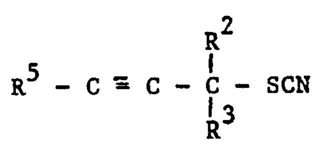

wherein :

$R^5$ is a hydrogen atom, a halogen atom, a hydrocarbyl group, a substituted hydrocarbyl group, a group $-CH_2SCN$ or a group $-SiR^2R^3R^4$ ; and

$R^2$, $R^3$ and $R^4$ are independently a hydrogen atom, a hydrocarbyl group or a substituted hydrocarbyl group, with the proviso that when the group $R^5$ is a hydrogen atom, a methyl group, a phenyl group or a group $-CH_2SCN$, at least one of the groups $R^2$ or $R^3$ is a hydrocarbyl group or a substituted hydrocarbyl group.

New compounds in accordance with the present invention include those in which the groups $R^2$ and $R^3$ are both hydrogen and the group $R^5$ is a halogen atom, especially bromine or iodine ; an alkyl group containing at least two carbon atoms, for example an n-butyl or n-heptyl group ; a halocarbon group such as a chloromethyl, bromomethyl or trifluoromethyl group ; a substituted alkyl group such as a hydroxymethyl group, an acetoxymethyl group or a cyanoethoxymethyl group ; or a silyl group such as a trimethylsilyl group. Other compounds in accordance with the present invention include those in which $R^5$ is a hydrogen atom and at least one of $R^2$ and $R^3$ is an alkyl group, such as a methyl group.

The compounds in accordance with the present invention have anti-fungal activity together with some activity against bacteria and preferred compounds show both anti-fungal activity and anti-bacterial activity. The preferred compounds of the present invention are those in which $R^5$ is, or contains, at least one halogen atom, or is a group $-SiR^2R^3R^4$. Especially preferred compounds are those in which $R^5$ is a halogen atom, for example iodopropargyl thiocyanate and bromopropargyl thiocyanate.

The propargyl derivatives may be prepared by known procedures, for example as described in Ber. (1873) 6, 729 ; Chem.Pharm.Bull.Jap. (1962) 10, 1094 and Rec.Trav.Chim. (1974) 93, 29.

A convenient method of preparing the thiocyanate derivative is by the reaction of the corresponding

propargyl halide with an alkali metal thiocyanate or ammonium thiocyanate.

The reaction may be effected in any suitable solvent such as, for example, a lower alkanol, an aqueous lower alkanol, a ketone such as acetone, N,N-dimethylformamide, N-methylpyrrolidone, glyme, diglyme and cellosolve.

The reaction is preferably effected at a relatively low temperature, for example, not more than 80°C, which may be ambient temperature or below for example 15°C. If the reaction is effected at a temperature above ambient temperature, it is conveniently effected in acetone under reflux, that is at a temperature between 55 and 60°C.

Some propargyl halides, for example propargyl bromide and propargyl chloride are commercially available materials. However, if desired, the desired propargyl halide can be obtained by the reaction of the corresponding alcohol with a halogenating agent, such as, for example phosphorus tribromide, phosphorus pentachloride, and thionyl chloride. The reaction is preferably effected in the presence of a base, particularly an organic base such as pyridine. The halogenating reaction is preferably effected in a liquid medium which is a solvent for the alcohol and preferably also for the halide product. The reaction is preferably effected at a temperature not exceeding 80°C and especially not exceeding 50°C. Conveniently the reactants are mixed at a temperature which is initially below ambient temperature, for example from 0°C to 10°C.

The halide may be purified before reaction with the thiocyanate compound but propargyl thiocyanates can be obtained by reaction of the thiocyanate compound with the halide reaction mixture, without isolating and purifying the halide.

The desired propargyl thiocyanate can be isolated and purified using any suitable technique. Thus, the propargyl thiocyanate may be recrystallised from a suitable solvent or solvent mixture, for example from a mixture of methylene chloride and a low boiling petroleum ether fraction. Alternatively, the propargyl thiocyanate may be purified by a chromatographic technique, for example by flash chromatography.

Further aspects of the present invention are described in the following illustrative examples.

In the following examples, the products obtained were subjected to microbiostatic evaluation and some products were also subjected to microbiocidal evaluation. The microbiological testing was effected, under sterile conditions throughout, as follows :

Preparation of Inoculum

Bacteria

The bacterial inoculum consisted of 24 hour cultures of the organisms grown in Oxoid Nutrient Broth, subcultured daily and incubated at 37°C.

Fungi

Spore suspensions of each of the test fungi were prepared as follows. To 250 $cm^3$ conical flasks containing well sporulating cultures of the organisms, growing on Oxoid Malt Extract agar, a number of sterile 3 mm glass beads and approximately 50 $cm^3$ of a sterile solution of 0.01% v/v of polyoxyethylene (20) sorbitan mono-oleate (available from Imperial Chemical Industries PLC as Tween 80) (Tween is a Registered Trade Mark) in water were added. Each flask was swirled so that the beads removed the spores and the resulting suspension was poured into a sterile 100 g medical flat bottle containing approximately 50 $cm^3$ of the sterile 0.01% v/v solution of Tween 80. The suspension could be stored for up to four weeks at 4°C.

In the microbiological testing, the products were tested for anti-microbial activity against bacteria and/or fungi. The bacteria used were one or more of Escherichia coli, Staphylococcus aureus, and Pseudomonas aeruginosa. The fungi used were one or more of Aspergillus niger, Aureobasidum pullulans, Cladosporium sphaerospermum, Aspergillus versicolor, and Chaetomium globosum.

These test organisms will be referred to hereafter as EC, SA, PA, AN, AP, CS, AV and CG respectively.

Microbiostatic evaluation

Method A

0.3 g of the product to be tested was dissolved in 3.0 $cm^3$ of N,N-dimethylformamide to give a 10% w/v solution.

For each of the products being tested, five bottles containing 50 $cm^3$ of Oxoid Malt agar and five bottles containing 50 $cm^3$ of Oxide Nutrient agar were heated by steam to melt the contents. The bottles were cooled

to 50°C and a sufficient quantity of the solution of the product was added to give a concentration of the product in the agar of 1 ppm, 5 ppm, 25 ppm, 125 ppm or 625 ppm. The lower concentrations of product were obtained by diluting the initial 10% w/v solution to 1% w/v or 1000 ppm and adding the diluted solution to the melted agar. From each bottle treated as described, two petri dish plates were poured and allowed to set overnight.

The test organisms were surface inoculated onto the test plates by means of a multi-point inoculator.

The test plates obtained from malt agar were incoulated with fungi and the plates were incubated at 25°C for five days.

The test plates obtained from nutrient agar were inoculated with bacteria and the plates were incubated for 24 hours at 37°C.

At the end of the incubation period, the plates were assessed visually for growth of the micro-organisms. The concentration of the product which inhibited the growth of a particular micro-organism was recorded.

Method B

100 mg of the product to be tested was dissolved in 2 $cm^3$ of N,N-dimethylformamide and the solution obtained diluted with a further quantity of N,N-dimethylformamide to give a product concentration of 2500 ppm.

To bottles containing 50 $cm^3$ of Czapek Dox agar containing 0.5% v/v peptone at 50°C was added a quantity of the product solution to give a concentration of 500 ppm or 25 ppm of the product. From each bottle, two petri dish plates were poured and allowed to set overnight.

The test organisms were surface inoculated onto the test plates by means of a multi-point inoculator. Each test plate was inoculated with both bacteria and fungi. The plates were incubated for four days at 25°C.

At the end of the incubation period, the plates were assessed visually for growth of the micro-organisms. The concentration of the product which inhibited the growth of a particular micro-organism was recorded.

Microbiocidal Evaluation

The product to be tested was added to 100 $cm^3$ of sterile distilled water contained in 250 $cm^3$ conical flasks to give concentrations of 1 ppm, 5 ppm, 25 ppm, 125 ppm and 250 ppm. The flasks were equilibrated to 37°C in a shaking water bath. A sample of an overnight culture of EC was added to give an initial test inoculum of about $10^7$ CFU/$cm^3$.

After periods of one hour and four hours, a one $cm^3$ sample of the test liquid is removed from the flask and added to 9 $cm^3$ of a sterile inactivating liquid consisting of 2% of polyoxyethylene (20) sorbitan monooleate (Tween 80), 3% of azolectin and 95% of Oxoid Nutrient broth. The solution thus obtained was diluted by factors of 10, 100 and 1000 with saline solution. All four concentration levels were enumerated for viable survivors by pour plate technique. The test plates were incubated at 37°C for 48 hours and the plates counted to determine which showed a kill of at least 99.99%. The concentration of product which gave a four decimal reduction (that is 99.99% kill) compared to a control flask, which did not contain the product under test, was noted.

Example 1

Potassium iodide (3.8 g), iodine (5.9 g) and methanol (40 $cm^3$) were stirred together at 15°C for 15 minutes. A solution of propargyl bromide (available from Fluka) (2.8 g) in methanol (10 $cm^3$) was added to this mixture and the resulting mixture was stirred for a further 10 minutes at 15°C. A solution of potassium hydroxide (1.6 g) in methanol (10 $cm^3$) was added over a period of 15 minutes whilst maintaining the temperature at 10-13°C and the reaction mixture was stirred for a further 20 minutes at 15°C. Potassium thiocyanate (2.5 g) was added in portions whilst maintaining the temperature at 15°C and the reaction mixture was then stirred for a further two hours, allowing it to warm up to ambient temperature (21°C). The reaction mixture was screened and evaporated to dryness using a rotary evaporator at a bath temperature of below 40°C with a vacuum obtained using a water pump. The residue, which was an oil which partly solidified when ice water (100 $cm^3$) was added, was extracted into methylene chloride (100 $cm^3$). The methylene chloride solution was washed with water (100 $cm^3$), dried over anhydrous magnesium sulphate, screened through a short bed of silica gel and evaporated to dryness using a rotary evaporator at a bath temperature of below 40°C with a vacuum obtained using a water pump. The residual oil thus obtained readily solidified when triturated with light petroleum (b.pt. 60-80°C) and was recrystallised by dissolving in methylene chloride, adding petroleum ether (b.pt. 40-60°C) dropwise until precipitation commenced and allowing to

crystallise to give 2.2 g of very pale yellow soft crystals m.pt. 58-61°C. The infra-red spectrum (KBr disc) showed absorption peaks at 3000 $cm^{-1}$ and 2950 $cm^{-1}$ (both characteristic of $-CH_2-$), 2180 $cm^{-1}$ and 2140 $cm^{-1}$ (characteristic of C=C and -SCN respectively). However, the spectrum did not contain peaks at 3290 $cm^{-1}$ (characteristic of H-C=), 2050 $cm^{-1}$ (characteristic of -NCS) or at 1980 $cm^{-1}$ (characteristic of C=C=C).

By analysis the composition was found to be C 21.5% wt ; H 0.5% wt ; N 6.2% wt ; I 57.3% wt and S 14.5% wt. $C_4H_2INS$ requires C 21.5% wt ; H 0.9% wt ; N 6.3% wt ; I 61.5% wt and S 14.3% wt.

Example 2

The compound of Example 1 was evaluated against a range of bacteria and fungi by the microbiological techniques hereinbefore described. In microbiostatic evaluation, using Method A, control for the test organisms was obtained at the following levels :

| | |
|---|---|
| EC | 5 ppm |
| SA | 5 ppm |
| PA | 5 ppm |
| AN | 1 ppm |
| AP | 1 ppm |
| CS | 1 ppm |
| AV | 1 ppm |
| CG | 1 ppm |

In the microbiocidal evaluation, this compound caused 4 decimal reductions (99.99% kill) in the culture of EC in the times, and at the levels, set out hereafter :

| | |
|---|---|
| 1 hour | 125-625 ppm |
| 4 hours | 25-125 ppm |

In all of the following examples, parts are by weight with the exception of solvents where parts are by volume.

Example 3

4 Parts of propargyl bromide were dissolved in 60 parts of a 75 : 25 v/v methanol/water mixture and the mixture was cooled, with stirring, to -5°C. A solution of 4 parts of potassium hydroxide in 20 parts of the 75 : 25 water/methanol mixture were added dropwise to the stirred solution. The resulting mixture was cooled to -10°C and 5.4 parts of bromine dissolved in 15 parts of the 75 : 25 methanol/water mixture were added dropwise over a period of 20 minutes whilst continuing to stir the mixture at -5°C to -10°C. Stirring was continued for a further 20 minutes at -5°C to -10°C and the mixture was then neutralised to a pH of 6 by the dropwise addition of aqueous 2N hydrochloric acid at a temperature of -5°C to 5°C.

2.2 Parts of potassium thiocyanate dissolved in 12 parts of the 75 : 25 methanol/water mixture were added to the neutralised solution and the resulting mixture was stirred for one hour at 5°C to 10°C. A further 1.8 parts of potassium thiocyanate were added and the reaction mixture was stirred overnight and allowed to warm up to ambient temperature. The reaction mixture was screened and evaporated to dryness, as in Example 1, and the oily residue was purified by flash chromatography on Kieselgel 60 (a silica gel available from Merck GmbH of Darmstadt, Germany). Elution was effected using petroleum ether (b.pt. 60-80°C) with increasing proportions of chloroform. 1-Bromopropargyl-3-thiocyanate was obtained as a colourless oil. The infra-red spectrum obtained using a thin film of the oil showed peaks characteristic of acetylene (2210 $cm^{-1}$) and thiocyanate (2150 $cm^{-1}$) groups. By analysis the composition was found to be C 26.7% wt ; H 0.6% wt; N 7.5% wt ; Br 45.8% wt and S 18.5% wt. $C_4H_2BrNS$ requires C 27.3% wt ; H 1.1% wt ; N 8.0% wt ; Br 45.5% wt and S 18.2% wt.

In microbiostatic evaluation, using Method A, the compound provided control of the test organisms as follows :

| | |
|---|---|
| EC | 1 ppm |
| SA | 1 ppm |
| PA | 1 ppm |
| AN | 1 ppm |
| AP | 1 ppm |
| CS | 1 ppm |
| AV | 1 ppm |
| CG | 1 ppm |

In the microbiocidal evaluation, 5 ppm of this compound caused 4 decimal reductions in the culture of

EC within one hour.

Example 4

10 Parts of dec-2-yn-1-ol (available from Lancaster Synthesis), 1 part of dry pyridine and 25 parts of dry diethyl ether were stirred at a temperature in the range 0 to 5°C whilst 6.6 parts of phosphorus tribromide were added dropwise. The mixture was heated, with stirring, up to reflux temperature and maintained under reflux conditions for three hours. The mixture was cooled to a temperature in the range 0 to 5°C and diluted with a further 75 parts of diethyl ether. The diluted mixture was then repeatedly extracted with small portions of ice cold 5% w/v brine saturated with sodium bicarbonate. The treated ether fraction was dried using anhydrous magnesium sulphate, and the ether distilled off under a water pump vacuum and at a temperature of below 40°C. The residual product was purified by distillation under a water pump vacuum in a Kugelrohr to give 8 parts of 1-bromodec-2-yne as a colourless oil having an absorption peak at 2230 $cm^{-1}$, which is characteristic of the acetylenic group.

4.34 Parts of the 1-bromodec-2-yne were added to a stirred solution containing 2.2 parts of potassium thiocyanate in 50 parts of a 90 : 10 v/v ethanol/water mixture. The resulting mixture was stirred at ambient temperature for four days. The reaction mixture was screened and evaporated to dryness as in Example 1. The residual oil was purified by flash chromatography as in Example 1. A yield of 3 parts of 1-thiocyanatodec-2-yne as a colourless oil was obtained.

By analysis the composition was found to be C 67.6% wt ; H 8.4% wt ; N 7.0% wt ; and S 17.0% wt. $C_{11}H_{17}NS$ requires C 67.6% wt ; H 8.7% wt ; N 7.2% wt ; and S 16.5% wt. The infra-red spectrum, using a thin film of the oil, showed peaks characteristic of acetylene (2230 $cm^{-1}$) and thiocyanate (2150 $cm^{-1}$) groups. However, peaks corresponding to isothiocyanate and allene groups were not observed.

In microbiostatic evaluation using Method A, the compound provided control of the test organisms as follows :

AN 25 ppm
AP 25 ppm
CS 25 ppm
AV 5 ppm
CG 5 ppm

Example 5

The procedure described in Example 4 was repeated using hept-2-yn-1-ol (available from Lancaster Synthesis) to obtain 1-thiocyanatohept-2-yne as a colourless oil. The infra-red spectrum, using a thin film of the oil, contained a strong thiocyanate (2150 $cm^{-1}$) peak.

In microbiostatic evaluation, using Method A, the compound provided control of the test organisms as follows :

EC 125 ppm
SA 125 ppm
AN 25 ppm
AP 25 ppm
CS 25 ppm
AV 25 ppm
CG 25 ppm

At the highest level tested (625 ppm), control of PA was not achieved.

Example 6

The procedure described in Example 4 was repeated using 3-phenylprop-2-yn-1-ol (available from Lancaster Synthesis) to obtain 1-thiocyanato-3-phenylprop-2-yne as a colourless oil. The infra-red spectrum contained a strong thiocyanate (2150 $cm^{-1}$) peak.

In microbiostatic evaluation, using Method A, the compound provided control of the test organisms as follows :

EC 125 ppm
PA 625 ppm
SA 125 ppm
AN 1 ppm

AP 1 ppm
CS 1 ppm
AV 1 ppm
CG 1 ppm

Example 7

The procedure described in Example 4 was repeated using but-2-yn-1-ol (available from Lancaster Synthesis) to obtain 1-thiocyanatobut-2-yne as a colourless oil. The infra-red spectrum contained a strong thiocyanate (2150 $cm^{-1}$) peak.

In microbiostatic evaluation, using Method A, the compound provided control of the test organisms as follows :

EC 625 ppm
PA 625 ppm
SA 625 ppm
AN 1 ppm
AP 1 ppm
CS 1 ppm
AV 1 ppm
CG 1 ppm

Example 8

Using the procedure described in Ber. (1873), 6, 729, 1-thiocyanatoprop-2-yne was prepared from propargyl bromide and potassium thiocyanate in alcohol. The product was confirmed to have the thiocyanate structure from the infra-red absorption spectrum (peak at 2150 $cm^{-1}$, no peak at 2050 $cm^{-1}$).

In microbiostatic evaluation, using Method A, the compound provided control of the test organisms as follows :

EC 125 ppm
SA 125 ppm
AN 1 ppm
AP 1 ppm
CS 1 ppm
AV 1 ppm
CG 1 ppm

At the highest level tested (625 ppm), control of PA was not achieved.

Example 9

Using the procedure described in JCS (1946), 1009, 1,4-dichlorobut-2-yne was prepared from but-2-yn-1,4-diol (available from Lancaster Synthesis).

2 Parts of 1,4-dichlorobut-2-yne were stirred for two hours under reflux conditions with 6.25 parts of ammonium thiocyanate and 50 parts of acetone. The mixture was cooled. The cooled mixture was then screened and evaporated to dryness as in Example 1. The residual oil was purified by flash chromatography (as in Example 3) to give 1,4-dithiocyanatobut-2-yne as a colourless oil. The infra-red spectrum using a thin film of the oil contained the peak characteristic of the thiocyanato group (2150 $cm^{-1}$). Proton magnetic resonance using $CDCl_3$ as solvent and tetramethylsilane as internal reference showed a singlet peak at a delta value of 3.8 ppm, this being characteristic of hydrogen in $-C{\equiv}CCH_2SCN$.

By analysis the composition was found to be C 42.6% wt ; H 2.3% wt ; N 16.5% wt ; and S 37.5% wt. $C_6H_4N_2S_2$ requires C 42.9% wt ; H 2.4% wt ; N 16.7% wt ; and S 38.1% wt.

In microbiostatic evaluation, using Method A, the product provided control of the organisms as follows:

EC 1 ppm
PA 1 ppm
SA 1 ppm
AN 5 ppm
AP 5 ppm
CS 5 ppm
AV 5 ppm

CG 5 ppm

Example 10

Using the procedure described in JACS (1955), 77, 166, 4-chlorobut-2-yn-1-ol was prepared from but-2-yn-1,4-diol (available from Lancaster Synthesis).

2.1 parts of 4-chlorobut-2-yn-1-ol were stirred for two hours under reflux conditions with 6.25 parts of ammonium thiocyanate and 50 parts of acetone and the mixture was then cooled. The cool mixture was screened and evaporated to dryness as in Example 1. The residual oil was purified by flash chromatography (as in Example 3) to give 4-thiocyanatobut-2-yn-1-ol as a colourless oil. The infra-red spectrum using a thin film of the oil contained the peak characteristic of the thiocyanato group (2160 $cm^{-1}$) and also a broad peak at 3400 $cm^{-1}$ (hydroxyl group). The pmr spectrum (obtained as described in Example 9) contained triplet peaks at delta values of 3.85 ppm and 4.3 ppm (both due to $-CH_2-$) and a peak at 4.1 ppm (due to the labile proton in OH).

By analysis the composition was found to be C 47.8% wt ; H 3.7% wt ; N 10.7% wt ; and S 26.3% wt. $C_5H_5NOS$ requires C 47.2% wt ; H 3.9% wt ; N 11.0% wt ; and S 25.2% wt.

In microbiostatic evaluation, using Method A, the product provided control of the test organisms as follows :

PA 625 ppm
AN 625 ppm
AP 125 ppm
CS 125 ppm
AV 125 ppm
CG 125 ppm

At the highest level tested (625 ppm), control of EC and SA was not achieved.

Example 11

Acetylation of the product of Example 10 was effected using boron trifluoride and acetic anhydride at ambient temperature. The infra-red spectrum of 4-acetoxy-1-thiocyanatobut-2-yne contained the peaks characteristic of the thiocyanato group (2160 $cm^{-1}$) and the carbonyl

$$( >C = O)$$

group (1740 $cm^{-1}$).

In microbiostatic evaluation, using Method B, the product provided control of the test organisms as follows :

EC 500 ppm
PA 500 ppm
AN 25 ppm
AP 25 ppm
CS 25 ppm
AV 25 ppm
CG 25 ppm

Lower levels than 25 ppm were not tested.

Example 12

4.25 parts of 1,4-dibromobut-2-yne (obtained from Fairfield Chemicals) and 42 parts of acetone were stirred at ambient temperature to obtain a homogeneous solution. 1.52 parts of ammonium thiocyanate were added and the mixture was stirred at ambient temperature for three days. The mixture was screened and evaporated to dryness as in Example 1. The residual oil was subjected to flash chromatography (as in Example 3) and separated into 1 part of unchanged starting material, 0.7 parts of 1,4-dithiocyanatobut-2-yne (found to be identical with the product of Example 9) and 1.5 parts of 1-bromo-4-thiocyanatobut-2-yne. The infra-red spectrum of 1-bromo-4-thiocyanatobut-2-yne contained the characteristic peak of the thiocyanato group (2155 $cm^{-1}$). The pmr spectrum (obtained as described in Example 9) contained peaks at delta values of 3.85 ppm and 4.02 ppm. These peaks were barely resolved triplets and were attributed to the hydrogens

present in the groups $-CH_2SCN$ and $-CH_2Br$ respectively.

In microbiostatic evaluation, using Method B, the product provided control of the test organisms as follows :

EC 25 ppm
PA 25 ppm
AN 25 ppm
AP 25 ppm
CS 25 ppm
AV 25 ppm
CG 25 ppm

Levels lower than 25 ppm were not tested.

Example 13

Using the procedure described in Org.Syn. 64, 182, 3-trimethylsilylprop-2-yn-1-ol was prepared from propargyl alcohol.

Using the procedure of Example 4, the bromo derivative was obtained using phosphorus tribromide, pyridine and diethyl ether.

6.3 Parts of 1-bromo-3-trimethylsilylprop-2-yne were stirred for 18 hours at ambient temperature with 3.65 parts of ammonium thiocyanate in 25 parts of acetone. The reaction mixture was then screened, evaporated to dryness and purified by flash chromatography as described in Example 3. The infra-red spectrum contained absorption peaks characteristic of methyl, acetylene and thiocyanato groups (2960 $cm^{-1}$, 2180 $cm^{-1}$ and 2160 $cm^{-1}$ respectively). The pmr spectrum (obtained as described in Example 9) contained peaks at delta values of 0.2 ppm (due to methyl hydrogen in the trimethylsilyl group) and 3.7 ppm (hydrogen in $-CH_2SCN$).

In microbiostatic evaluation, using Method B, the product provided control of the test organisms as follows :

PA 25 ppm
AN 25 ppm
AP 25 ppm
CS 25 ppm
AV 25 ppm
CG 25 ppm

Levels lower than 25 ppm were not tested.

Example 14

1.25 Parts of 1,4-dichlorobut-2-yne (obtained as described in Example 9) were stirred for four hours under reflux conditions with 0.76 parts of ammonium thiocyanate and 25 parts of acetone. The mixture was cooled, screened, evaporated to dryness and purified by flash chromatography as described in Example 9 to give 0.2 parts of 1,4-dithiocyanatobut-2-yne (found to be identical with the product of Example 9) and 0.39 parts of 1-thiocyanato-4-chlorobut-2-yne as a colourless oil. The infra-red spectrum of 1-thiocyanato-4-chlorobut-2-yne contained the characteristic peak of the thiocyanato group (2160 $cm^{-1}$). The pmr spectrum (obtained as described in Example 9) contained triplets at delta values of 3.8 ppm (hydrogen of the $-CH_2SCN$ group) and 4.2 ppm (hydrogen of the $-CH_2Cl$ group).

In microbiostatic evaluation, using Method B, the product provided control of the test organisms as follows :

EC 25 ppm
PA 25 ppm
AN 25 ppm
AP 25 ppm
CS 25 ppm
AV 25 ppm
CG 25 ppm

Levels lower than 25 ppm were not tested.

Example 15

Using the procedure of Example 4, 2.78 parts of 4-(2-cyanoethoxy)but-2-yn-1-ol were reacted with 1.7 parts of phosphorus tribromide, 0.25 parts of pyridine and 7 parts of dry diethyl ether. The bromo derivative was isolated from the reaction mixture as in Example 4 but was not subjected to a distillation step to purify the product.

2.3 parts of the crude 4-(2-cyanoethoxy)-1-bromobut-2-yne was stirred overnight (about 17 hours) at ambient temperature with 1 part of ammonium thiocyanate and 50 parts of acetone. The reaction mixture was then screened, evaporated to dryness and purified by flash chromatography as described in Example 3. The product, 1-(2-cyanoethoxy)-4-thiocyanatobut-2-yne, was obtained as a colourless oil, the infra-red spectrum of which contained the characteristic peaks of the cyano and thiocyanato groups (2250 $cm^{-1}$ and 2160 $cm^{-1}$ respectively).

In microbiostatic evaluation, using Method B, the product provided control of the test organisms as follows :

EC 500 ppm
PA 500 ppm
AN 25 ppm
AP 25 ppm
CS 25 ppm
AV 25 ppm
CG 25 ppm

Levels lower than 25 ppm were not tested.

Example 16

The procedure of Example 7 was repeated using but-1-yn-3-ol (available from Lancaster Synthesis) to obtain 3-thiocyanatobut-1-yne. The infra-red spectrum contained a peak characteristic of the hydrogen atom attached to the acetylene group and a peak characteristic of the thiocyanato group (3290$cm^{-1}$ and 2150$cm^{-1}$ respectively).

In microbiostatic evaluation, using Method B, the compound provided control of the test organisms as follows :

EC 500 ppm
PA 500 ppm
AN 25 ppm
AP 25 ppm
CS 25 ppm
AV 25 ppm
CG 25 ppm

Levels lower than 25 ppm were not tested.

**Claims**

1. A biocide composition which is a solution, suspension or emulsion in water of at least one compound of the formula :

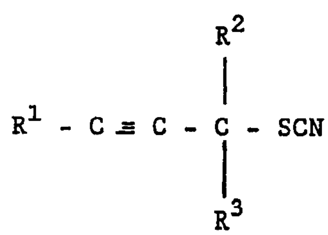

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the sub-

stituted hydrocarbyl group $R^1$ is other than a carbonate group and is a hydrocarbyl group, an acyloxy group, one or more halogen atoms, a nitrile group, a hydroxyl group, an ether group or a substituted ether group, where the substituent of the substituted ether group is a hydrocarbyl group, an acyloxy group, one or more halogen atoms, a nitrile group, a hydroxyl group or an ether group.

2. A composition as claimed in claim 1 wherein $R^1$ is a hydrogen atom, a phenyl group, an alkyl group containing not more than 10 carbon atoms, or a group $-SiR^2R^3R^4$.

3. A composition as claimed in claim 1 wherein $R^1$ is a group $-CH_2SCN$ or is, or contains, a halogen atom.

4. A method for inhibiting the growth of micro-organisms on, or in, a medium, which comprises treating the medium with at least one compound of the formula :

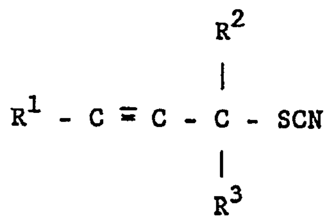

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is a hydrocarbyl group, an ester group, one or more halogen atoms, a nitrile group, a hydroxyl group, an ether group or a substituted ether group, where the substituent of the substituted ether group is a hydrocarbyl group, an ester group, one or more halogen atoms, a nitrile group, a hydroxyl group or an ether group.

5. A method as claimed in claim 4 wherein the medium which is treated is a cooling water system, a paper mill liquor, a metal working fluid, a geological drilling lubricant, a polymer emulsion, a paint, a lacquer, a varnish, leather or wood.

6. A medium which is selected from a cooling water system, a paper mill liquor, a metal working fluid, a geological drilling lubricant, a polymer emulsion, a paint, a varnish, a lacquer, which contains at least one compound of the formula :

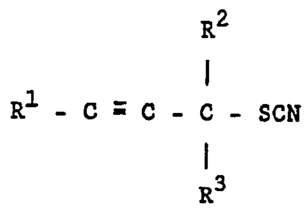

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is a hydrocarbyl group, an ester group, one or more halogen atoms, a nitrile group, a hydroxyl group, an ether group or a substituted ether group, where the substituent of the substituted ether group is a hydrocarbyl group, an ester group, one or more halogen atoms, a nitrile group, a hydroxyl group or an ether group.

7. Leather or wood which has been impregnated or coated with a medium which is, or which contains, at least one compound of the formula :

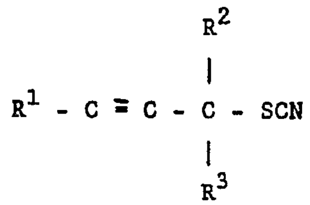

wherein :

R¹ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is a hydrocarbyl group, an ester group, one or more halogen atoms, a nitrile group, a hydroxyl group, an ether group or a substituted ether group, where the substituent of the substituted ether group is a hydrocarbyl group an ester group, one or more halogen atoms, a nitrile group, a hydroxyl group or an ether group.

8. A compound of the formula :

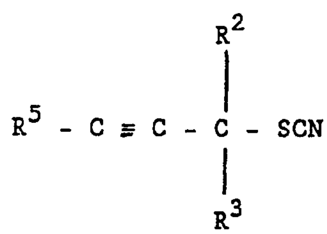

wherein :

$R^5$ is a hydrogen atom, a halogen atom, a hydrocarbyl group, a substituted hydrocarbyl group, a group $-CH_2SCN$ or a group $-SiR^2R^3R^4$ ; and

$R^2$, $R^3$ and $R^4$ are independently a hydrogen atom or a methyl group, with the proviso that

when the group $R^5$ is a methyl group, a hydroxymethyl group, a phenyl group, a group $-CH_2SCN$, or a group $-Si(CH_3)_3$, at least one of the groups $R^2$ or $R^3$ is a methyl group ;

when the group $R^5$ is a hydrogen atom both $R^2$ and $R^3$ are other than a hydrogen atom ;

when the group $R^5$ is $-C(CH_3)_2Cl$ or $-C(CH_3)(C_2H_5)C1$, $R^2$ and $R^3$ are not both methyl groups ; and

when the group $R^5$ is $-Si(CH_3)_3$ and one of $R^2$ and $R^3$ is methyl, the other of $R^2$ and $R^3$ is other than hydrogen ;

and the substituent of the substituted hydrocarbyl group $R^5$ is other than a carbonate group and is at least one group selected from hydrocarbyl groups, acyloxy groups, halogen atoms, nitrile groups, hydroxy groups, ether groups or substituted ether groups, where the substituent of the substituted ether groups is a hydrocarbyl group, an acyloxy group, one or more halogen atoms, a nitrile group, a hydroxyl group or an ether group.

9. A compound as claimed in claim 8 which is
iodopropargylthiocyanate,
bromopropargylthiocyanate,
1-thiocyanatodec-2-yne,
1-thiocyanatohept-2-yne,
4-acetoxy-1-thiocyanatobut-2-yne,
1-bromo-4-thiocyanatobut-2-yne,
1-thiocyanato-4-chlorobut-2-yne,
1-(2-cyanoethoxy)-4-thiocyanatobut-2-yne.

10. A compound of the formula :

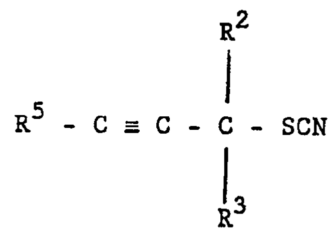

wherein :

$R^5$ is a halogen atom, a hydrocarbyl group other than a methyl or phenyl group or a substituted hydrocarbyl group other than a hydroxymethyl group or a hydrocarbon substituted chloromethyl group : and $R^2$, and $R^3$ are independently a hydrogen atom or a methyl group wherein the substituent of the substituted hydrocarbyl group $R^5$ is other than a carbonate group and is at least one group selected from hydrocarbyl groups, acyloxy groups, halogen atoms, nitrile groups, hydroxyl groups, ether groups or substituted ether groups, where the substituent of the substituted ether group is a hydrocarbyl group, an acyloxy group, one or more halogen atoms, a nitrile group, a hydroxyl group or an ether group.

**Claims for ES**

1. A biocide composition which is a solution, suspension or emulsion in water of at least one compound of the formula :

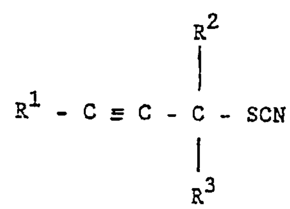

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is other than a carbamate group and is a hydrocarbyl group, an acyloxy group, one or more halogen atoms, a nitrile group, a hydroxyl group, an ether group or a substituted ether group, where the substituent of the substituted ether group is a hydrocarbyl group, an acyloxy group, one or more halogen atoms, a nitrile group, a hydroxyl group or an ether group.

2. A composition as claimed in claim 1 wherein $R^1$ is a hydrogen atom, a phenyl group, an alkyl group containing not more than 10 carbon atoms, or a group $-SiR^2R^3R^4$.

3. A composition as claimed in claim 1 wherein $R^1$ is a group $-CH_2SCN$ or is, or contains, a halogen atom.

4. A method for inhibiting the growth of micro-organisms on, or in, a medium, which comprises treating the medium with at least one compound of the formula :

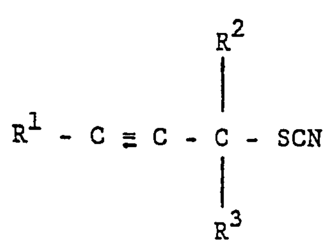

wherein

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is a hydrocarbyl group, an ester group, one or more halogen atoms, a nitrile group, a hydroxyl group, an ether group or a substituted ether group, where the substituent of the substituted ether group is a hydrocarbyl group, an ester group, one or more halogen atoms, a nitrile group, a hydroxyl group or an ether group.

5. A method as claimed in claim 4 wherein the medium which is treated is a cooling water system, a paper mill liquor, a metal working fluid, a geological drilling lubricant, a polymer emulsion, a paint, a lacquer, a varnish, leather or wood.

6. A method as claimed in either claim 4 or claim 5 wherein the compound used is one in which $R^1$ is a hydrogen atom, a phenyl group, an alkyl group containing not more than 10 carbon atoms, or a group $-SiR^2R^3R^4$.

7. A method as claimed in either claim 4 or claim 5 wherein the compound used is one in which the group $R^1$ is a group $-CH_2SCN$ or is, or contains, at least one halogen atom.

8. A method as claimed in either claim 4 or claim 5 wherein the compound used is
iodopropargylthiocyanate,
bromopropargylthiocyanate,
1-thiocyanatodec-2-yne,
1-thiocyanatohept-2-yne,
1-thiocyanato-3-phenylprop-2-yne,
1-thiocyanatobut-2-yne,
1-thiocyanatoprop-2-yne,
1,4-dithiocyanatobut-2-yne,
4-thiocyanatobut-2-yn-1-ol,
4-acetoxy-1-thiocyanatobut-2-yne,
1-bromo-4-thiocyanatobut-2-yne,
1-thiocyanato-3-trimethylsilylprop-2-yne,
1-thiocyanato-4-chlorobut-2-yne,
1-(2-cyanoethoxy)-4-thiocyanatobut-2-yne, or
3-thiocyanatobut-1-yne.

9. A medium which is selected from a cooling water system, a paper mill liquor, a metal working fluid, a geological drilling lubricant, a polymer emulsion, a paint, a varnish, or a lacquer, which contains at least one compound of the formula :

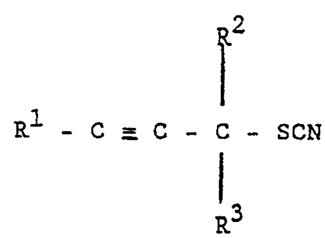

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is a hydrocarbyl group, an ester group, one or more halogen atoms, a nitrile group, a hydroxyl group, an ether group or a substituted ether group, where the substituent of the substituted ether group is a hydrocarbyl group, an ester group, one or more halogen atoms, a nitrile group, a hydroxyl group or an ether group.

10. Leather or wood which has been impregnated or coated with a medium which is, or which contains, at least one compound of the formula :

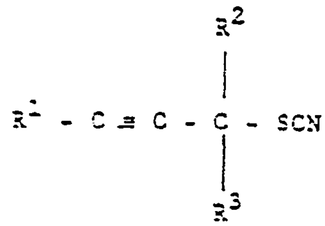

wherein :

$R^1$ is a hydrogen or a halogen atom, a hydrocarbyl group, a substituted hydrocarbyl group, a $-CH_2SCN$ group or a group $-SiR^2R^3R^4$ ; and

$R^2$, $R^3$ and $R^4$ are independently a hydrogen atom or a methyl group wherein the substituent of the substituted hydrocarbyl group $R^1$ is a hydrocarbyl group, an ester group, one or more halogen atoms, a nitrile group, a hydroxyl group, an ether group or a substituted ether group, where the substituent of the substituted ether group is a hydrocarbyl group, an ester group, one or more halogen atoms, a nitrile group, a hydroxyl group or an ether group.

## Ansprüche

### Ansprüche für Vertreagstraten BE, CH, DE, FR, GB, IT, LI, LU, NL, SE.

1. Biocide Zusammensetzung, bestehend aus einer Lösung, Suspension oder Emulsion mindestens einer Verbindung der folgenden Formel in Wasser :

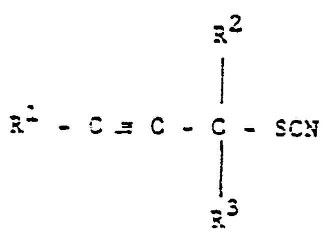

worin

$R^1$ für ein Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ mit Ausnahme einer Carbamat-Gruppe aus einer Kohlenwasserstoff-Gruppe, einer Acyloxy-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe aus einer Kolhlenwasserstoff-Gruppe, einer Acyloxy-Gruppe, einem oder mehreren Halogen-Atomen, einer Nitril-Gruppe, einer Hydroxyl-Gruppe oder einer Ether-Gruppe besteht.

2. Zusammensetzung nach Anspruch 1, worin $R^1$ für ein Wasserstoff-Atom, eine Phenyl-Gruppe, eine Alkyl-Gruppe mit nicht mehr als 10 Kohlenstoff-Atomen oder eine $-SiR^2R^3R^4$ -Gruppe steht.

3. Zusammensetzung nach Anspruch 1, worin $R^1$ für eine $-CH_2SCN$ -Gruppe steht oder ein Halogen-Atom ist oder ein solches enthält.

4. Verfahren zur Verhinderung des Wachstums von Mikro-organismen auf oder in einem Medium, bei welchem das Medium mit mindestens einer Verbindung der Formel

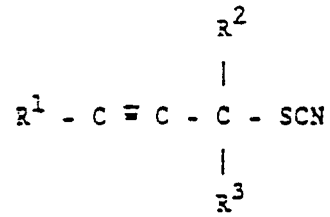

behandelt wird, worin

$R^1$ für Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ aus einer Kohlenwasserstoff-Gruppe, einer Ester-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe aus einer Kohlenwasserstoff-Gruppe, einer Ester-Gruppe, einem oder mehreren Halogen-Atomen, einer Nitril-Gruppe, einer Hydroxyl-Gruppe oder einer Ether-Gruppe besteht.

5. Verfahren nach Anspruch 4, bei welchem das behandelte Medium ein Kühlwassersystem, eine Papiermühlenflüssigkeit, eine Metallbearbeitungsflüssigkeit, ein geologisches bohrschmiermittel, eine Polymeremulsion, eine Anstrichfarbe, ein Lack, ein Firnis, Leder oder Holz ist.

6. Medium, welches ausgewählt ist aus einem Kühlwassersystem, einer Papiermühlenflüssigkeit, einer Metallbearbeitungsflüssigkeit, einem geologischen Bohrschmiermittel, einer Polymeremulsion, einer Anstrichfarbe, einem Firnis oder einem Lack und welches mindestens eine Verbindung der Formel

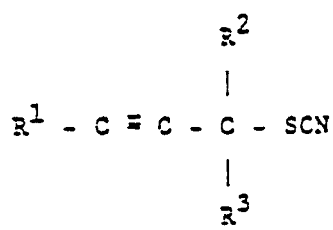

enthält, worin

$R^1$ für ein Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ aus einer Kohlenwasserstoff-Gruppe, einer Ester-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe aus einer Kohlenwasserstoff-Gruppe, einer Ester-Gruppe, einem oder mehreren Halogen-Atomen, einer Nitril-Gruppe, einer Hydroxy-Gruppe oder einer Ether-Gruppe besteht.

7. Leder oder Holz, welches mit einem Medium imprägniert oder beschichtet worden ist, das mindestens eine Verbindung der Formel

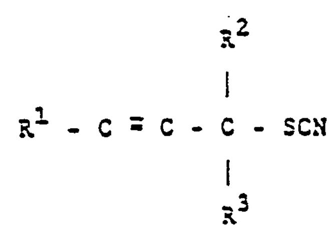

ist oder eine solche Verbindung enthält, worin

$R^1$ für ein Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ aus einer Kohlenwasserstoff-Gruppe, einer Ester-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe aus einer Kohlenwasserstoff-Gruppe, einer Ester-Gruppe, einem oder mehreren Halogen-Atomen, einer Nitril-Gruppe, einer Hydroxy-Gruppe oder einer ether-Gruppe besteht.

8. Verbindung der Formel

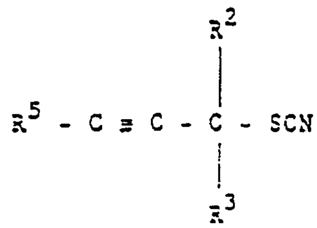

worin

$R^5$ für ein Wasserstoff-Atom, ein Halogen-Atom, eine Kohlenwasserstoff-Gruppe, eine substituierte Kohlenwasserstoff-Gruppe, eine $-CH_2SCN$ -Gruppe oder $-SiR^2R^3R^4$ -Gruppe steht und

$R^2$, $R^3$ und $R^4$ unabhängig für ein Wasserstoff-Atom oder eine Methyl-Gruppe stehen, mit der Maßgabe, daß,

wenn die Gruppe $R^5$ eine Methyl-Gruppe, eine Hydroxymethyl-Gruppe, eine Phenyl-Gruppe, eine $-CH_2SCN$ -Gruppe oder eine $-Si(CH_3)_3$ -Gruppe ist, mindestens eines der Symbole $R^2$ oder $R^3$ für eine Methyl-Gruppe steht,

wenn die Gruppe $R^5$ ein Wasserstoff-Atom ist, $R^2$ und $R^3$ beide für etwas anderes als ein Wasserstoff-Atom stehen,

wenn die Gruppe $R^5$ $-C(CH_3)_2Cl$ oder $-C(CH_3)(C_2H_5)Cl$ ist, $R^2$ und $R^3$ nicht beide für Methyl-Gruppen stehen, und

wenn die Gruppe $R^5$ $-Si(CH_3)_3$ ist und eines der Symbole $R^2$ und $R^3$ für Methyl steht, das andere der Symbole $R^2$ und $R^3$ für etwas anderes als Wasserstoff steht,

wobei die Substitution in der substituierten Kohlenwasserstoff-Gruppe $R^5$ mit Ausnahme einer Carbamat-Gruppe aus mindestens einer Gruppe besteht, die ausgewählt ist aus Kohlenwasserstoff-Gruppen, Acyloxy-Gruppen, Halogen-Atomen, Nitril-Gruppen, Hydroxy-Gruppen, Ether-Gruppen oder substituierten Ether-Gruppen und die Substitution in der substituierten Ether-Gruppe aus einer Kohlenwasserstoff-Gruppe, einer Acyloxy-Gruppe, einem oder mehreren Halogen-Atomen, einer Nitril-Gruppe, einer Hydroxyl-Gruppe oder einer Ether-Gruppe besteht.

9. Verbinbung nach Anspruch 8, welche eine der folgenden ist:

Jodopropargylthiocanat,
Bromopropargylthiocyanat,
1-Thiocyanatodec-2-in,
1-Thiocyanatohept-2-in,
4-Acetoxy-1-thiocyanatobut-2-in,
1-Bromo-4-thiocyanatobut-2-in,
1-Thiocyanato-4-chlorobut-2-in und
1-(2-Cyanoethoxy)-4-thiocyanatobut-2-in.

10. Verbindung der Formel

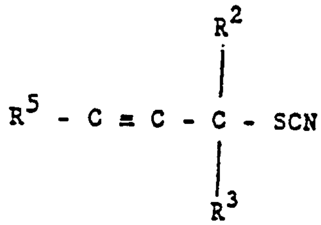

worin

$R^5$ für ein Halogen-Atom, eine Kohlenwasserstoff-Gruppe mit Ausnahme einer Methyl- oder Phenyl-Gruppe oder eine substituierte Kohlenwasserstoff-Gruppe mit Ausnahme einer Hydroxymethyl-Gruppe oder eine durch Kohlenwasserstoff substituierte Chloromethyl-Gruppe steht und

$R^2$ und $R^3$ unabhängig für ein Wasserstoff-Atom oder eine Methyl-Gruppe stehen, wobei die Substitution in der substituierten Kohlenwasserstoff-Gruppe $R^5$ mit Ausnahme einer Carbamat-Gruppe aus mindestens einer Gruppe besteht, die ausgewählt ist aus Kohlenwasserstoff-Gruppen, Acyloxy-Gruppen, Halogen-Atomen, Nitril-Gruppen, Hydroxyl-Gruppen, Ether-Gruppen oder substituierten Ether-Gruppen, und die Substitution in der substituierten Ether-Gruppe aus einer Kohlenwasserstoff-Gruppe, einer Acyloxy-Gruppe, einem oder mehreren Halogen-Atomen, einer Nitril-Gruppe, einer Hydroxyl-Gruppe oder einer Ether-Gruppe besteht.

## Ansprüche ES

1. Biocide Zusammensetzung, bestehend aus einer Lösung, Suspension oder Emulsion mindestens einer Verbindung der folgenden Formel in Wasser :

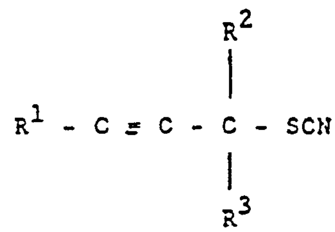

worin

$R^1$ für ein Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ mit Ausnahme einer Carbamat-Gruppe aus einer Kohlenwasserstoff-Gruppe, einer Acyloxy-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe, aus einer Kohlenwasserstoff-Gruppe, einer Acyloxy-Gruppe, einem oder mehreren Halogen-Atomen, einer Nitril-Gruppe, einer Hydroxyl-Gruppe oder einer Ether-Gruppe besteht.

2. Zusammensetzung nach Anspruch 1, worin $R^1$ für ein Wasserstoff-Atom, eine Phenyl-Gruppe, eine Alkyl-Gruppe mit nicht mehr als 10 Kohlenstoff-Atomen oder eine $-SiR^2R^3R^4$ -Gruppe steht.

3. Zusammensetzung nach Anspruch 1, worin $R^1$ für eine $-CH_2SCN$ -Gruppe steht oder ein Halogen-Atom ist oder ein solches enthält.

4. Verfahren zur Verhinderung des Wachstums von Mikro-organismen auf oder in ein Medium, bei welchem das Medium mit mindestens einer Verbindung der Formel

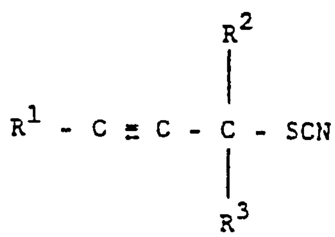

behandelt wird, worin

$R^1$ für ein Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ aus einer Kohlenwasserstoff-Gruppe, einer Ester-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe aus einer Kohlenwasserstoff-Gruppe, einer Acyloxy-Gruppe, einem oder mehreren Halogen-Atomen, einer Nitril-Gruppe, einer Hydroxyl-Gruppe oder einer Ether-Gruppe besteht.

5. Verfahren nach Anspruch 4, bei welchem das behandelte Medium ein Kühlwassersystem, eine Papiermühlenflüssigkeit, eine Metallbearbeitungsflüssigkeit, ein geologisches Bohrschmiermittel, eine Polymeremulsion, eine Anstrichfarbe, ein Lack, ein Firnis, Leder oder Holz ist.

6. Verfahren nach einem der Ansprüche 4 oder 5, bei welchem die verwendete Verbindung eine solche ist, in welcher $R^1$ für ein Wasserstoff-Atom, eine Phenyl-Gruppe, eine Alkyl-Gruppe mit nicht mehr als 10 Kohlenstoff-Atomen oder eine $-SiR^2R^3R^4$ -Gruppe steht.

7. Verfahren nach einem der Ansprüche 4 oder 5, bei welchem die verwendete Verbindung eine solche ist, in welcher die Gruppe $R^1$ für eine $-CH_2SCN$ -Gruppe steht oder mindestens ein Halogen-Atom ist oder ein solches enthält.

8. Verfahren nach einem der Ansprüche 4 oder 5, bei welchem die verwendete Verbindung eine der folgenden ist:
Jodopropargylthiocyanat,
Bromopropargylthiocyanat,
1-Thiocyanatodec-2-in,
1-Thiocyanatohept-2-in,
1-Thiocyanato-3-phenylprop-2-in,
1-Thiocyanatobut-2-in,
1-Thiocyanatoprop-2-in,
1,4-Dithiocyanatobut-2-in,
4-Thiocyanatobut-2-in-1-ol,
4-Acetoxy-1-thiocyanatobut-2-in,
1-Bromo-4-thiocyanatobut-2-in,
1-Thiocyanato-3-trimethylsilylprop-2-in,
1-Thiocyanato-4-chlorobut-2-in,
1-(2-Cyanoethoxy)-4-thiocyanatobut-2-in oder
3-Thiocyanatobut-1-in.

9. Medium, welches ausgewählt ist aus einem Kühlwassersystem, einer Papiermühlenflüssigkeit, einer Metallbearbeitungsflüssigkeit, einem geologischen Bohrschmiermittel, einer Polymeremulsion, einer Anstrichfarbe, einem Firnis oder einem Lack und welches mindestens eine Verbindung der Formel

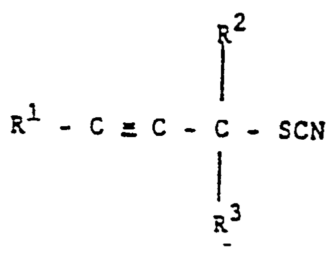

enthält, worin

$R^1$ für ein Wasserstoff- oder Halogen-Atom, eine Kohlenwasserstoff-Gruppe, eine substituierte Kohlenwasserstoff-Gruppe, eine $-CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ aus einer Kohlenwasserstoff-Gruppe, einer Ester-Gruppe, einem oder mehreren Halogen-Atomen, einer Nitril-Gruppe, einer Hydroxyl-gruppe, einer Ether-Gruppe oder einer substituierten Ether-Gruppe besteht und die Substitution in der substituierten Ether-Gruppe aus einer Kohlenwasserstoff-Gruppe, einer Ester-Gruppe, einem oder mehreren Halogen-Atomen, einer Nitril-Gruppe, einer Hydroxyl-Gruppe oder einer Ether-Gruppe besteht.

10. Leder oder Holz, welches mit einem Medium imprägniert oder beschichtet worden ist, das mindestens eine Verbindung der Formel

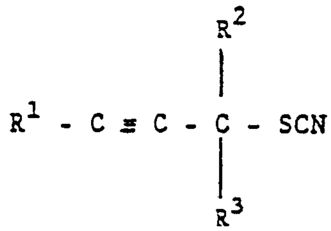

ist oder eine solche Verbindung enthält, worin

$R^1$ für ein Wasserstoff- oder Halogen-Atom, eine Kohlenwasserstoff-Gruppe, eine substituierte Kohlenwasserstoff-Gruppe, eine $CH_2SCN$ -Gruppe oder eine $-SiR^2R^3R^4$ -Gruppe steht und

$R^2$, $R^3$ und $R^4$ unabhängig für ein Wasserstoff-Atom oder eine Methyl-Gruppe stehen, wobei die Substitution in der substituierten Kohlenwasserstoff-Gruppe $R^1$ aus einer Kohlenwasserstoff-Gruppe, einer Ester-Gruppe, einem oder mehreren Halogen-Atomen, einer Nitril-Gruppe, einer Hydroxyl-Gruppe, einer Ether-Gruppe oder einer substituierten Ether-Gruppe besteht und die Substitution in der substituierten Ether-Gruppe aus einer Kohlenwasserstoff-Gruppe, einer Ester-Gruppe, einem oder mehreren Halogen-Atomen, einer Nitril-Gruppe, einer Hydroxyl-Gruppe oder einer Ether-Gruppe besteht.

**Revendications**

**Revendications pour BE, CH, DE, FR, GB, GR, IT, LI, LU, NL, SE.**

1. Composition biocide qui est une solution, suspension ou émulsion dans de l'eau d'au moins un composé de formule :

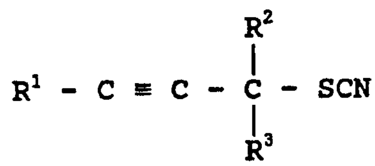

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est autre qu'un radical carbamate et est un radical hydrocarbyle, un radical acyloxy, un ou plusieurs atomes d'halogène, un radical nitrile, un radical hydroxyle, un radical éther ou un radical éther substitué, où le substituant du radical éther substitué est un radical hydrocarbyle, un radical acyloxy, un ou plusieurs atomes d'halogène, un radical nitrile, un radical hydroxyle ou un radical éther.

2. Composition suivant la revendication 1, dans laquelle $R^1$ est un atome d'hydrogène, un radical phényle, un radical alcoyle ne comptant pas plus de 10 atomes de carbone ou un radical $-SiR^2R^3R^4$.

3. Composition suivant la revendication 1, dans laquelle $R^1$ est un radical $-CH_2SCN$ ou est, ou contient, un atome d'halogène.

4. Procédé pour inhiber la croissance de micro-organismes sur ou dans un milieu, qui comprend le traitement de ce milieu à l'aide d'au moins un composé de formule :

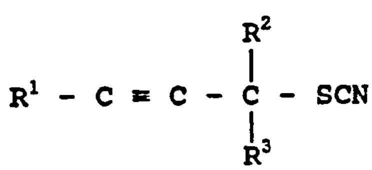

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est un radical hydrocarbyle, un radical ester, un ou plusieurs atomes d'halogène, un radical nitrile, un radical hydroxyle, un radical éther ou un radical éther substitué, où le substituant du radical éther substitué est un radical hydrocarbyle, un radical ester, un ou plusieurs atomes d'halogène, un radical nitrile, un radical hydroxyle ou un radical éther.

5. Procédé suivant la revendication 4, dans lequel le milieu qui est traité est un système d'eau de refroidissement, une eau de papeterie, un liquide pour le travail des métaux, un lubrifiant de forage géologique, une émulsion de polymère, une peinture, une laque, un vernis, le cuir ou le bois.

6. Milieu choisi parmi un système d'eau de refroidissement, une eau de papeterie, un liquide pour le travail des métaux, un lubrifiant de forage géologique, une émulsion de polymère, une peinture, un vernis, et une laque, qui contient au moins un composé de formule :

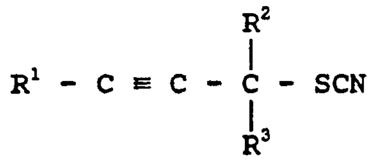

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est un radical hydrocarbyle, un radical ester, un ou plusieurs atomes d'halogène, un radical nitrile, un radical hydroxyle, un radical éther ou un radical éther substitué, où le substituant du radical éther substitué est un radical hydrocarbyle, un radical ester, un ou plusieurs atomes d'halogène, un radical nitrile, un radical hydroxyle ou un radical éther.

7. Cuir ou bois qui a été imprégné ou revêtu d'un milieu qui est, ou contient, au moins un composé de formule :

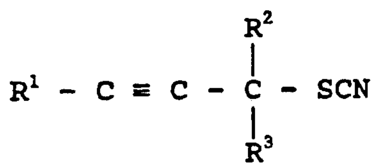

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est un radical hydrocarbyle, un radical ester, un ou plusieurs atomes d'halogène, un radical nitrile, un radical hydroxyle, un radical éther ou un radical éther substitué, où le substituant du radical éther substitué est un radical hydrocarbyle, un radical ester, un ou plusieurs atomes d'halogène, un radical nitrile, un radical hydroxyle ou un radical éther.

8. Composé de formule :

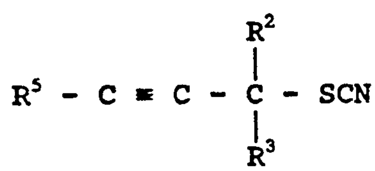

où

$R^5$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, avec la restriction que

lorsque $R^5$ est un radical méthyle, unradical hydroxyméthyle, un radical phényle, un radical $-CH_2SCN$ ou un radical $-Si(CH_3)_3$, au moins l'un des radicaux $R^2$ et $R^3$ est un radical méthyle ;

lorsque le radical $R^5$ est un atome d'hydrogène, $R^2$ et $R^3$ sont tous deux autres qu'un atome d'hydro-

gène ;

lorsque le radical $R^5$ est $-C(CH_3)_2Cl$ ou bien $-C(CH_3)(C_2H_5)Cl$, $R^2$ et $R^3$ ne sont pas tous deux des radicaux méthyle, et

lorsque le radical $R^5$ est $-Si(CH_3)_3$ et que l'un d'entre $R^2$ et $R^3$ est méthyle, l'autre d'entre $R^2$ et $R^3$ est autre qu'hydrogène ;

et le substituant du radical hydrocarbyle substitué $R^5$ est autre qu'un radical carbamate et est au moins un radical choisi parmi les radicaux hydrocarbyle, les radicaux acyloxy, les atomes d'halogène, les radicaux nitrile, les radicaux hydroxyle, les radicaux éther et les radicaux éther substitués, où le substituant des radicaux éther substitués est un radical hydrocarbyle, un radical acyloxy, un ou plusieurs atomes d'halogène, un radical nitrile, un radical hydroxyle ou un radical éther.

9. Composé suivant la revendication 8 qui est :

le thiocyanate d'iodopropargyle,
le thiocyanate de bromopropargyle,
le 1-thiocyanatodéc-2-yne,
le 1-thiocyanatohept-2-yne,
le 4-acétoxy-1-thiocyanatobut-2-yne,
le 1-bromo-4-thiocyanatobut-2-yne,
le 1-thiocyanato-4-chlorobut-2-yne,
le 1-(2-cyanoéthoxy)-4-thiocyanatobut-2-yne.

10. Composé de formule :

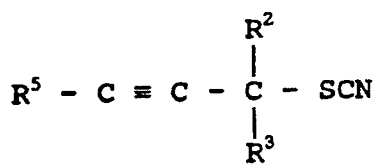

où

$R^5$ est un atome d'halogène, un radical hydrocarbyle autre qu'un radical méthyle ou phényle ou un radical hydrocarbyle substitué autre qu'un radical hydroxyméthyle ou un radical chlorométhyle hydrocarbyl substitué, et

$R^2$ et $R^3$ sont indépendamment un atome d'hydrogène ou un radical méthyle où le substituant du radical hydrocarbyle substitué $R^5$ est autre qu'un radical carbamate et est au moins un radical choisi parmi les radicaux hydrocarbyle, les radicaux acyloxy, les atomes d'halogène, les radicaux nitrile, les radicaux hydroxyle, les radicaux éther et les radicaux éther substitués, où le substituant du radical éther substitué est un radical hydrocarbyle, un radical acyloxy, un ou plusieurs atomes d'halogène, un radical nitrile, un radical hydroxyle ou un radical éther.

**Revendications pour ES**

1. Composition biocide qui est une solution, suspension ou émulsion dans de l'eau d'au moins un composé de formule :

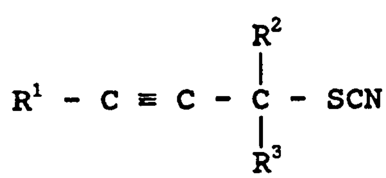

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est autre qu'un radical carbamate et est un radical hydrocarbyle, un radical acyloxy, un ou plusieurs atomes d'halogène, un radical nitrile, un radical hydroxyle, un radical éther ou un radical éther substitué, où le substituant du radical éther substitué est un radical hydrocarbyle, un radical acyloxy, un ou plusieurs atomes d'halogène, un radical nitrile, un radical hydroxyle ou

un radical éther.

2. Composition suivant la revendication 1, dans laquelle $R^1$ est un atome d'hydrogène, un radical phényle, un radical alcoyle ne comptant pas plus de 10 atomes de carbone ou un radical $-SiR^2R^3R^4$.

3. Composition suivant la revendication 1, dans laquelle $R^1$ est un radical $-CH_2SCN$ ou est, ou contient, un atome d'halogène.

4. Procédé pour inhiber la croissance de micro-organismes sur ou dans un milieu, qui comprend le traitement de ce milieu à l'aide d'au moins un composé de formule :

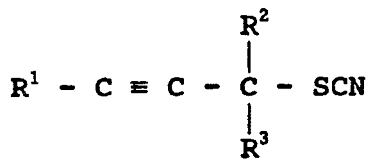

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est un radical hydrocarbyle, un radical ester, un ou plusieurs atomes d'halogène, un radical nitrile, un radical hydroxyle, un radical éther ou un radical éther substitué, où le substituant du radical éther substitué est un radical hydrocarbyle, un radical ester, un ou plusieurs atomes d'halogène, un radical nitrile, un radical hydroxyle ou un radical éther.

5. Procédé suivant la revendication 4, dans lequel le milieu qui est traité est un système d'eau de refroidissement, une eau de papeterie, un liquide pour le travail des métaux, un lubrifiant de forage géologique, une émulsion de polymère, une peinture, une laque, un vernis, le cuir ou le bois.

6. Procédé suivant la revendication 4 ou 5, dans lequel le composé utilisé est un composé dans lequel $R^1$ est un atome d'hydrogène, un radical phényle, un radical alcoyle ne comptant pas plus de 10 atomes de carbone ou un radical $-SiR^2R^3R^4$.

7. Procédé suivant la revendication 4 ou 5, dans lequel le composé utilisé est un composé dans lequel $R^1$ est un radical $-CH_2SCN$ ou est, ou contient, un atome d'halogène.

8. Procédé suivant la revendication 4 ou 5, dans lequel le composé utilisé est :
le thiocyanate d'iodopropargyle,
le thiocyanate de bromopropargyle,
le 1-thiocyanatodéc-2-yne,
le 1-thiocyanatohept-2-yne,
le 1-thiocyanato-3-phénylprop-2-yne,
le 1-thiocyanatobut-2-yne,
le 1-thiocyanatoprop-2-yne,
le 1,4-dithiocyanatobut-2-yne,
le 4-thiocyanatobut-2-yn-1-ol,
le 4-acétoxy-1-thiocyanatobut-2-yne,
le 1-bromo-4-thiocyanatobut-2-yne,
le 1-thiocyanato-3-triméthylsilylprop-2-yne,
le 1-thiocyanato-4-chlorobut-2-yne,
le 1-(2-cyanoéthoxy)-4-thiocyanatobut-2-yne, ou
le 3-thiocyanatobut-1-yne.

9. Milieu choisi parmi un système d'eau de refroidissement, une eau de papeterie, un liquide pour le travail des métaux, un lubrifiant de forage géologique, une émulsion de polymère, une peinture, un vernis, et une laque, qui contient au moins un composé de formule :

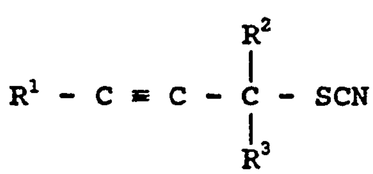

où

$R^1$ est un atome d'hydrogène, un atome d'halogène, un radical hydrocarbyle, un radical hydrocarbyle

substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est un radical hydrocarbyle, un radical ester, un ou plusieurs atomes d'halogène, un radical nitrile, un radical hydroxyle, un radical éther ou un radical éther substitué, où le substituant du radical éther substitué est un radical hydrocarbyle, un radical ester, un ou plusieurs atomes d'halogène, un radical nitrile, un radical hydroxyle ou un radical éther.

10. Cuir ou bois qui a été imprégné ou revêtu d'un milieu qui est, ou contient, au moins un composé de formule :

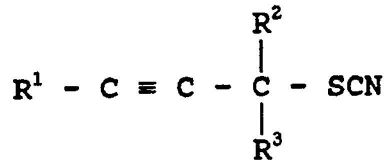

où

$R^1$ est un atome d'hydrogène, un atome d'halogène, un radical hydrocarbyle, un radical hydrocarbyle substitué, un radical $-CH_2SCN$ ou un radical $-SiR^2R^3R^4$ ; et

$R^2$, $R^3$ et $R^4$ sont indépendamment un atome d'hydrogène ou un radical méthyle, où le substituant du radical hydrocarbyle substitué $R^1$ est un radical hydrocarbyle, un radical ester, un ou plusieurs atomes d'halogène, un radical nitrile, un radical hydroxyle, un radical éther ou un radical éther substitué, où le substituant du radical éther substitué est un radical hydrocarbyle, un radical ester, un ou plusieurs atomes d'halogène, un radical nitrile, un radical hydroxyle ou un radical éther.